(19) Europäisches Patentamt
European Patent Office
Office européen des brevets

(11) **EP 3 681 453 B1**

(12) **EUROPEAN PATENT SPECIFICATION**

(45) Date of publication and mention
of the grant of the patent:
**25.10.2023 Bulletin 2023/43**

(21) Application number: **18779171.0**

(22) Date of filing: **11.09.2018**

(51) International Patent Classification (IPC):
**A61F 13/20** (2006.01)       **A61F 13/26** (2006.01)
**A61F 13/551** (2006.01)

(52) Cooperative Patent Classification (CPC):
**A61F 13/26; A61F 13/2051; A61F 13/2071;
A61F 13/55185**

(86) International application number:
**PCT/US2018/050369**

(87) International publication number:
**WO 2019/051455 (14.03.2019 Gazette 2019/11)**

(54) **DISPOSABLE ABSORBENT ARTICLE**

SAUGFÄHIGER EINWEGARTIKEL

ARTICLE ABSORBANT JETABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.09.2017   US 201762556667 P**

(43) Date of publication of application:
**22.07.2020   Bulletin 2020/30**

(60) Divisional application:
**22213029.6 / 4 169 493**

(73) Proprietor: **The Procter & Gamble Company
Cincinnati, OH 45202 (US)**

(72) Inventors:
• **VIENS, Gerard A.
Cincinnati
Ohio 45202 (US)**

• **STOEBELATHAM, Rebecca
Cincinnati
Ohio 45202 (US)**
• **MINOGUCHI, Ryo
Cincinnati
Ohio 45202 (US)**
• **QURESHI, Khalid
Cincinnati
Ohio 45202 (US)**
• **ALBACARYS, Alex
Cincinnati
Ohio 45202 (US)**

(74) Representative: **P&G Patent Germany
Procter & Gamble Service GmbH
Sulzbacher Straße 40
65824 Schwalbach am Taunus (DE)**

(56) References cited:
**US-A1- 2003 125 658       US-A1- 2014 142 232
US-A1- 2016 015 572       US-A1- 2017 020 743**

EP 3 681 453 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present disclosure pertains to disposable absorbent articles and more particularly to absorbent tampons which are inserted via a plastic applicator, wherein at least a portion of the materials of the absorbent article are sourced from renewable resources.

BACKGROUND OF THE INVENTION

Disposable absorbent articles are widely used throughout the world for various uses from baby diapers to bandages - as some examples. Many of these products utilize petrochemical based materials currently. Recently though there has been a push by society in general for products which utilize more renewable based materials.

Unfortunately, in many instances, the use of materials derived from renewable resources can pose processing challenges that did not exist with the utilization of their petrochemical / synthetic counterparts. One specific example in the realm of disposable absorbent articles pertains to tampons.

Several different varieties of tampons are currently available. Many utilize absorbents such as rayon or a combination of rayon and cotton. Some purport to use 100 percent cotton. However, the absorbent capacity of cotton - on a gram per gram basis - is less than that of rayon in the context of a tampon. As such, in order to achieve the same level of absorbency that one would get from a given mass of rayon, one would have to utilize more cotton to achieve that same level. US2003/0125658A1 discloses a tampon applicator assembly comprising up to 100% cotton as absorbent material. The additional mass of cotton can prove problematic, particularly when the tampon is inserted with an applicator.

While larger barrel applicators may be utilized to accommodate the larger mass of cotton absorbent, larger barrel applicators may provide some discomfort to the user. However, in order to fit the larger mass of cotton into the normal sized applicators, higher compression may be needed to force the cotton absorbent into a smaller diameter barrel. Unfortunately, higher compression can negatively impact the expansion of the absorbent. For example, an absorbent that has experienced too much compression does not readily expand upon fluid contact. The lack of expansion or decreased expansion can increase the likelihood of leakage experienced by the user.

Based on the above, there is a need for a disposable absorbent article that utilizes renewable materials that overcome the foregoing issues.

SUMMARY OF THE INVENTION

Absorbent articles of the present disclosure utilize renewable resources in their construction. Absorbent articles of the present disclosure comprise a plastic applicator and an absorbent tampon. The plastic applicator comprises a bio-based content of 10 percent to 100 percent using ASTM D6866-10, method B. The plastic applicator has an insertion portion and a plunger portion, the plunger portion being movable within the insertion portion. The absorbent tampon is disposed within the insertion portion. The absorbent tampon comprises a primary absorbent member. The primary absorbent member comprises from about 50 percent to about 100 percent by weight cotton fibers, and is compressed to a density ranging from 0.25 to 0.45 grams per cubic centimeter. The plunger portion is capable of expelling the absorbent tampon from the insertion portion.

BRIEF DESCRIPTION OF THE DRAWINGS

    Figure 1A is a schematic illustration showing an absorbent tampon in a tampon applicator.
    Figure 1B is a schematic illustration showing an insertion end of the tampon applicator of Figure 1A.
    Figure 2A is a schematic illustration showing a tampon pledget in its compressed state.
    Figures 2B-2D are schematic illustrations showing the tampon pledget of Figure 2A in its expanded state.
    Figure 3 is a schematic diagram showing an apparatus for measuring the dynamic expansion of tampons.
    Figure 4 is a schematic diagram of the apparatus of Figure 3 with some of the features of Figure 3 removed for ease of visualization.

DETAILED DESCRIPTION OF THE INVENTION

The following text sets forth a broad description of numerous different embodiments of the present invention. The description is to be construed as exemplary only and does not describe every possible embodiment since describing every possible embodiment would be impractical, if not impossible. And it will be understood that any feature, characteristic, component, composition, ingredient, product, step or methodology described herein can be deleted, combined with or substituted for, in whole or part, any other feature, characteristic, component, composition, ingredient, product, step or methodology described herein. Numerous alternative embodiments could be implemented, using either current technology or technology developed after the filing date of this patent, which would still fall within the scope of the claims.

"Array" means a display of packages comprising disposable articles of different sizes having like article constructions (e.g., same elastomeric materials [compositionally and/or structurally] in the flaps, graphic elements) said packages having the same brand and/or sub-brand, and said packages oriented in proximity to each other in a given area of a retail store. An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Depend," and same sub-brand, for example, "for Women Underwear." A different array may have the brand "Depend" and the sub-brand "Silhouette For Women." The differences between the "for Women Underwear" array and the "Silhouette For Women" arrays include different elastomeric materials in the side flaps, where "for Women Underwear" comprises strands as the elastomeric material and "Silhouette For Women" comprises a film elastomeric material." Furthermore, the packaging is distinctly different in that "for Women Underwear" is packaged in a predominately green, film bag and "Silhouette For Women" is packaged in a predominately maroon box.

Further regarding "Arrays," as another example of two separate "arrays" having the same brand, "Certainty," one line-up has the sub-brand "Women's Underwear." A different array may have the same brand "Certainty" and the sub-brand "Smooth Shape Briefs for Women." The differences between the "Women's Underwear" array and the "Smooth Shape Briefs for Women" arrays include different elastomeric materials in the side flaps, where "Women's Underwear" comprises strands as the elastomeric material and "Smooth Shape Briefs for Women" comprises a film elastomeric material." Furthermore, the packaging is distinctly different in that "Women's Underwear" is packaged in a predominately blue, film bag and "Smooth Shape Briefs for Women" is packaged in a predominately maroon box.

Arrays also often have the same trademarks, including trademarks of the brand, sub-brand, and/or features and/or benefits across the line-up.

"On-line Array" means an "Array" distributed by a common on-line source.

"Bio-based content" refers to the amount of carbon from a renewable resource in a material as a percent of the mass of the total organic carbon in the material, as determined by ASTM D6866-10, method B. Note that any carbon from inorganic sources such as calcium carbonate is not included in determining the bio-based content of the material.

"Renewable resource" refers to a natural resource that can be replenished within a 100 year time frame. The resource may be replenished naturally, or via agricultural techniques. Renewable resources include plants, animals, fish, bacteria, fungi, and forestry products. They may be naturally occurring, hybrids, or genetically engineered organisms. Natural resources such as crude oil, coal, and peat which take longer than 100 years to form are not considered to be renewable resources.

As used herein, "applicator" refers to a device or implement that facilitates the insertion of a feminine hygiene device, such as, e.g., a tampon or pessary, into an external orifice of a mammal. Exemplary applicators include telescoping, insertion portion and plunger, and compact applicators.

As used herein, the terms "pledget" and "tampon pledget" refer to a mass of absorbent material prior to compression of such material into tampon as described below. Tampon pledgets are sometimes referred to as a tampon blank, or a softwind.

As used herein, the term "tampon" refers to any type of absorbent structure which is inserted into the vaginal canal or other body cavities for the absorption of menses or other bodily exudates. Tampons typically are constructed from an absorbent material which has been compressed in any or all of the width direction, the radial direction, and the axial direction, in order to provide a tampon which is of a size and stability to allow insertion within the vagina or other body cavity. A tampon which has been so compressed is referred to herein as having a "self-sustaining" form. That is, the degree of compression and or conditioning of the absorbent material results in a tampon that will tend to retain its general shape and size prior to insertion into the body. The tampons of the present invention are "fluid-expanding" tampons that expand or uncompress upon contact and absorption of fluid. Fluid expanding tampons are contrasted to "mechanically expanding" tampons that may use springs or some other mechanical supplier of force to expand. An example of such a mechanically expanding tampon is described in U.S. Patent No. 3,706,311 to Kohx et al.

The "syngyna" test is an industry standard tampon absorbency capacity test. The apparatus and method for performing this tampon absorbency test are provided in 21 United States Code of Federal Regulation 801.430. In the United States and other regions, tampons carry standardized absorbency labels according to syngyna test absorbency ranges. Tampons absorbing 6 to 9 grams are labeled "regular absorbency," and tampons absorbing 9 to 12 grams are labeled "super absorbency."

As used herein, the term "transparent" means that a consumer can visually perceive what is on the opposite side of the transparent object or thing. The term transparent also includes translucent regions.

As used herein, the term "opaque" means that a consumer cannot visually perceive what is on the opposite side of the opaque object or thing.

Absorbent articles of the present invention comprise applicators and absorbent tampons therein. Either the applicators and/or the absorbent tampons comprise materials that are derived from renewable resources. Each of the applicator and absorbent tampon are described hereafter in additional detail.

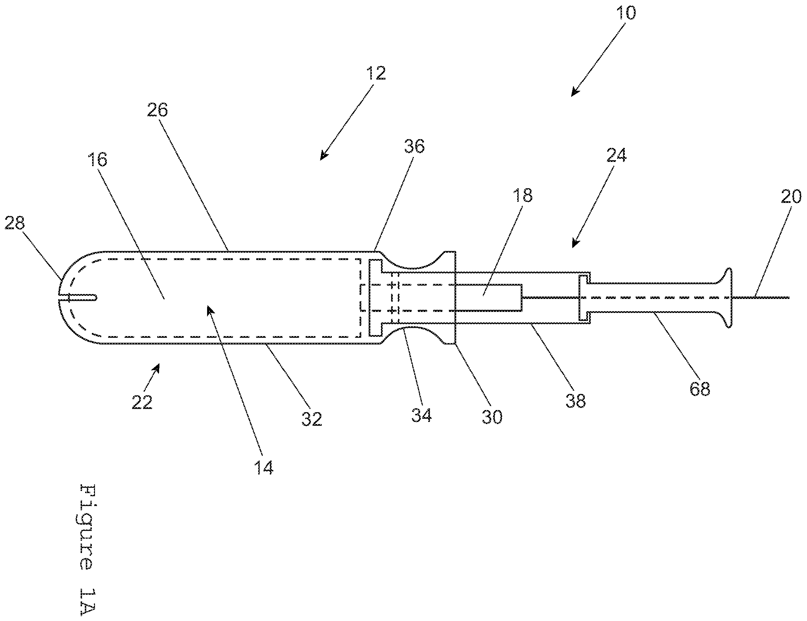

As shown in Figure 1A, an absorbent article 10 comprises an applicator 12 which houses an absorbent tampon 14. At least one of the applicator 12 and absorbent tampon 14 comprise materials that are derived from renewable resources as described herein.

The applicator 12 comprises an insertion portion 22 and a plunger portion 24. The insertion portion 22 comprises an outer surface 26 that defines an insertion end 28 and a withdrawal end 30, opposite the insertion end 28. The insertion end 28 is that portion of the applicator 12 that is intended to enter into the vaginal canal first when inserting the absorbent tampon 14 into the vaginal canal. The insertion portion 22 also comprises a barrel region 32 intermediate the insertion end 28 and the withdrawal end 30.

The plunger portion 24 can be operatively engaged with the insertion portion 22. Operatively engaged refers to the insertion portion 22 and the plunger portion 24 being configured for their intended purpose of housing and expelling the absorbent tampon 14 as is well known in the art. In one particular form of the present disclosure, the plunger portion 24 can comprise a first region 38 and a second region 68. In other forms, the plunger portion 24 may be a single unitary piece which is capable of expelling the tampon 14.

In some forms, the insertion portion 22 may comprise an indentation region 34. The indentation region 34 can extend inwardly from the outer surface 26 and can be disposed between the barrel region 32 and the withdrawal end 30. Stated another way, the indentation region 34 protrudes inward from the outer surface 26 of the insertion portion 22. The indentation region 34 can be disposed circumferentially around the outer surface 26 of the insertion portion 22. In addition, the indentation region 34 can be adjacent to a shoulder region 36. The shoulder region 36 can be disposed between the barrel region 32 and the indentation region 34. Generally, the shoulder region 36 refers to the area of the insertion portion 22 that slopes from the barrel region 32 to the indentation region 34. The indentation region 34 can be any shape that allows the consumer to grip the applicator 12. More specifically, for example, the indentation region 34 can be a concave shape or a square shape or some other shape having at least one of curved and straight portions.

In some forms, the indentation region 34 can comprise gripping formations. Additional features of the indention region and gripping formations can be found in U.S. Patent No. 8,449,491. Applicators having a grip region are further described in U.S. Pat. Nos. 8,303,558; 7,081,110; 8,449, 491; 8,075,512; or U.S. Patent Application No. 2017/0020743.

Now with reference to the plunger portion 24, in some forms, the plunger portion 24 may comprise a first region 38 and a second region 68. In such forms, the second region 68 may be engaged with the first region 38 such that the second region 68 may slide with respect to the first region 38. Additionally, either the first region 38 and/or the second region 68 may be transparent and/or opaque. In some particular forms, the first region 38 may be transparent while the second region 68 is opaque or vice versa. And, the second region 68 may be lockable with respect to the first region 38 via projections which are described in additional detail in U.S. Patent Application No. 2017/0020743.

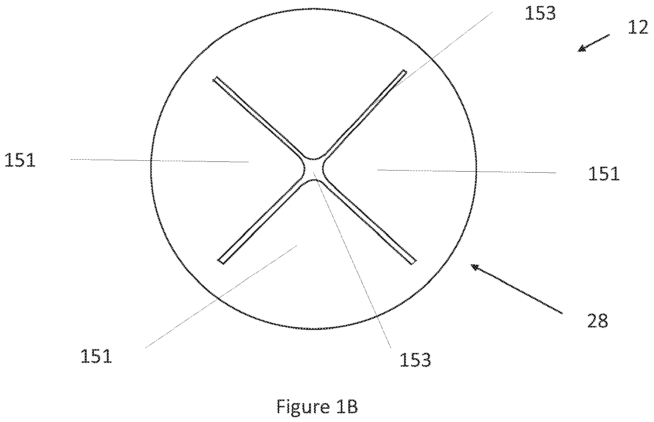

Referring now to Figures 1A and 1B, the insertion end 28 comprises a plurality of petals 151 with open space 153 between each of the adjacent petals. The petals 151 are flexible and allow the absorbent tampon 14 to be expelled from the applicator 12 during insertion of the absorbent tampon 14 into the vagina.

The applicator 12 or portions thereof, i.e. insertion portion 22 and/or plunger portion 24, can be constructed from a variety of polymers some of which, at least in part, may be derived from renewable resources. One particular example is low density polyethylene which is available from Braskem™ and sold under the trade names SPB208 and SPB608. Another suitable example is available from Chevron Phillips Chemical Co. and sold under the trade name Marlex™ KN226.

In some forms, both the insertion portion 22 and the plunger portion 24 may each comprise bio-based content. In some forms, only the insertion portion 22 may comprise bio-based content. In other forms, only the plunger portion 24 may comprise bio-based content. Either the insertion portion 22 and/or the plunger portion 24 may comprise a bio-based content value from about 10 percent to about 100 percent, from about 25 percent to about 90 percent, from about 40 percent to about 80 percent, or from about 50 percent to about 60 percent, specifically including all values within these ranges and any ranges created thereby.

The bio-based content value of the applicator 12 can be measured utilizing ASTM D6866-10, method B. In order to apply the methodology of ASTM D6866-10 to determine the bio-based content of the applicator, i.e. insertion portion and/or plunger portion, a representative sample of the applicator must be obtained for testing. In at least one particular form, the applicator or portion thereof, i.e. insertion portion and/or plunger portion, can be ground into particulates less than about 20 mesh using known grinding methods (e.g., Wiley® mill), and a representative sample of suitable mass taken from the randomly mixed particles.

In addition to the possible bio-based content of the applicator 12, forms are contemplated where the applicator 12 is substantially free of dyes and scents. For example, in some forms the applicator may comprise dyes and/or scents which modify the visual appearance and/or fragrance of the applicator. In some forms, the applicator may be free of such dyes and/or scents. Substantially free does not include trace amounts of color or fragrance which may be naturally occurring within the constituent materials utilized. The trace amounts of color or fragrance are typically present in less than 0.02 percent by weight. Other forms are contemplated where fragrance and/or odor management is provided via naturally-derived odor management materials.

For those forms where the applicator 12 is substantially free of dyes and scents, the applicator 12 may be translucent. Measures of translucency were obtained and are provided in Table 1.

Table 1

| Reference No. | Location | Barrel 1 | Barrel 2 | Barrel 3 |
|---|---|---|---|---|
| Sample #1 | 1 | 13.9 | 18.1 | 17.1 |
| Sample #1 | 2 | 14.3 | 15.3 | 15.6 |
| Sample #1 | 3 | 18.6 | 14.5 | 14.7 |
| Sample #2 | 1 | 15.3 | 16.5 | 16.1 |
| Sample #2 | 2 | 13.7 | 14.5 | 14.0 |
| Sample #2 | 3 | 12.5 | 13.9 | 11.0 |
| Conventional Example #5 | 1 | 34.4 | 31.9 | 36.5 |
| Conventional Example #5 | 2 | 35.3 | 34.7 | 35.2 |
| Conventional Example #5 | 3 | 34.6 | 31.9 | 34.0 |

Sample #1 was a super absorbency tampon constructed in accordance with the present description. Namely, the primary absorbent member comprises a 100% cotton and a 100% cotton overwrap. The applicator was created via bio-sourced polyethylene as described herein and comprised greater than about 90 percent bio-sourced polyethylene. Sample #2 was a regular absorbency tampon and constructed similar to Sample #1. Conventional Example #5 was available at CVS™ and sold under the trade name pure cotton tampons.

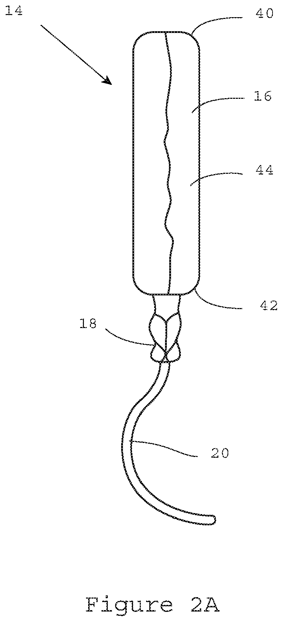

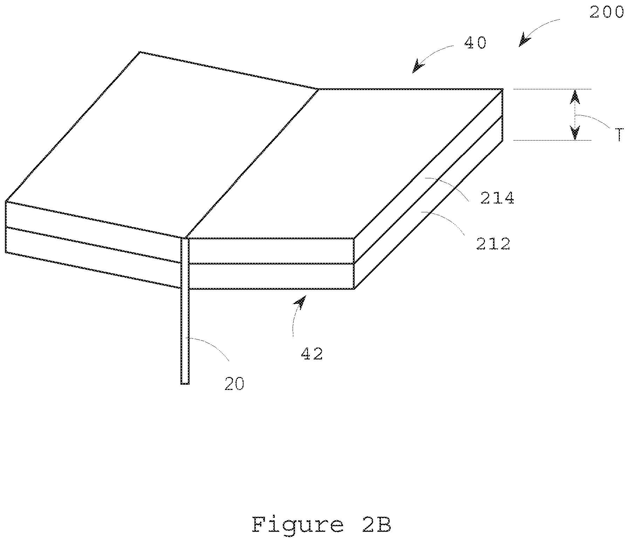

Similar to the applicator 12, in some forms, the absorbent tampon 14 within the applicator 12 may also comprise materials derived from renewable resources. Referring now to Figures 1A and 2A, the absorbent tampon 14 can comprise a primary absorbent member 16 having a leading end 40 and a trailing end 42, opposite the leading end 40. The primary absorbent member 16 can also comprise an intermediate region 44 between the trailing end 42 and the leading end 40. The primary absorbent member 16 can be formed from a tampon pledget (shown in Figures 2B-2D).

The absorbent tampon 14 may further comprise an overwrap which may be placed over some or all of the primary absorbent member 16. The overwrap may be liquid permeable material, if desired. The overwrap may be fluid wicking and may extend beyond the withdrawal end of the absorbent material to form a skirt portion as described in U.S. Patent No. 6,840,927, filed November 16, 2001, entitled "Tampon with Fluid Wicking Overwrap With Skirt Portion," issued to Hasse, et al. Typically, the overwrap may extend from about 2mm to about 30 mm beyond the withdrawal end of the primary absorbent member 16.

The fibers forming the overwrap may comprise any suitable material. In some forms, the fibers may be natural or derived from renewable resources, e.g. cotton. Or in some forms, the overwrap may be formed from synthetic fibers such as polyolefin fibers. Still in other forms, the overwrap may comprise a mix of natural fibers and synthetic fibers.

The fibers forming the overwrap may be made by any number of techniques. The blends may be carded on webs. Commonly, carded webs that are hydroentangled (spunlaced), thermally bonded, and resin bonded all have application. In the latter case, the resin bonding agent may be used in place of the synthetic fibers as the method for tempering the aggressiveness of the natural fiber matrix. In this case, all natural fiber may be used with a significant amount of synthetic binder (10-30% by weight is common). Spunbond and meltblown processes, combining synthetic fibers extruded/spun onto/into a mat or carded web of natural fibers provide other acceptable techniques. The basis weight of the overwrap may fall into a range from about 10, 12 or 15 grams per square meter to about 30, 40, 50 or 60 grams per square meter. The materials for the tampon may be formed into a fabric, web, or batt that is suitable for use in the pledget by any suitable process such as airlaying, carding, wetlaying, or other known techniques. Aperturing may be accomplished by any known method, such as by hydroentangling on a suitable forming screen, such as, for example, the method described in U.S. Pat. No. 3,025,585. Aperturing may also be accomplished by various processes involving bonding and stretching, such as those described in U.S. Pat. Nos. 3,949,127; 4,588,360; 5,873,868. The apertures may be zoned as described in U.S. Pat. No. 7,994,387, filed on October 17, 2007, entitled "Tampon having Zoned Apertured Overwrap," issued to Minoguchi, et al. In one embodiment, the apertures are formed by forming a plurality of spaced, melt stabilized regions, and then ring-rolling the web to stretch the web and form apertures in the melt stabilized regions, as described in U.S. Pat. Nos. 5,628,097 and 5,916,661.

Still referring to Figure 2A, in some forms, the absorbent tampon 14 may further comprise a secondary absorbent member 18 and/or a withdrawal member 20. As shown, the primary absorbent member 16 can be adjacent to or joined to at least one of a secondary absorbent member 18 and a withdrawal member 20. In one specific example, the secondary absorbent member 18 can be disposed within a portion of the intermediate region 44 of the primary absorbent member 16 and extend beyond the trailing end 42 of the primary absorbent member 16. In another example, the secondary absorbent member 18 can extend from the trailing end 42 of the primary absorbent member 16.

For those forms that include the secondary absorbent member 18, the secondary absorbent member 18 may be joined to the primary absorbent member 16 mechanically and/or chemically. For example, the secondary absorbent member 18 can be mechanically tied, such as by being knotted, stitched/sewn, or woven, such as by a braid, to the primary absorbent member 16. The secondary absorbent member 18 can also be chemically bonded to the primary absorbent member 16 by glue or other adhesives suitable for use in hygiene devices.

Still referring to Figure 2A, the tampon 14 can also comprise a withdrawal member 20. As shown, the withdrawal member 20 can extend from the secondary absorbent member 18. In some forms, the withdrawal member 20 can extend from the primary absorbent member 16. The withdrawal member 20 can be used to withdraw the tampon 14 post use, to aid during insertion of the tampon 14, and/or to offer reassurance of proper placement post insertion of the tampon 14. More specifically, for example, the withdrawal member 14 can be used by the consumer to signal that the tampon 14 has been properly loaded in the applicator 12 by extending beyond the end of the applicator 12, which will be discussed in more detail below. Further, the withdrawal member 20 can signal proper placement post insertion by remaining external of the vagina.

The withdrawal member 20 can be disposed on at least one of the primary absorbent member 16 and the secondary absorbent member 18. Stated another way, the withdrawal member 20 can be integral with or an extension of another part of the tampon 14, such as the primary absorbent member 16 or the secondary absorbent member 18. More specifically, the withdrawal member 20 can be attached, mechanically and/or chemically, to the primary absorbent member 16 and/or the secondary absorbent member 18. The withdrawal member 20 should be attached such that the tampon 14 can withstand use and post use removal. In one example embodiment, the withdrawal member 20 can be sewn to the primary absorbent member 16. In another example embodiment, the withdrawal member 20 can be interweaved, such as by braiding, with the secondary absorbent member 18.

Similar to the applicator, the primary absorbent member 16 may be substantially free of dyes and scents which modify the visual appearance of the primary absorbent member 16 and/or fragrance of the primary absorbent member 16. Additionally, in some forms, the secondary absorbent member may comprise dyes and/or scents which modify the visual appearance and/or fragrance of the secondary absorbent member. In some forms, the secondary absorbent member may be free of such dyes and/or scents. Other forms are contemplated where scent and/or odor control management can be achieved via naturally-derived odor management materials.

As mentioned previously, the absorbent tampon 14 may comprise material which is derived from renewable resources. One particular example is cotton. As such, forms of the present invention are contemplated wherein at least one of the primary absorbent member 16, the secondary absorbent member 18, and/or the withdrawal member 20 comprise material that is derived from renewable resources, e.g. cotton. For example, at least one of the primary absorbent material 16, the secondary absorbent material 18, and/or the withdrawal member 20, may comprise cotton in some particular forms. In some forms, the level of cotton by weight may be greater than about 60 percent, greater than about 75 percent, greater than about 90 percent, greater than about 95 percent, or 100 percent, specifically including any values within these ranges and any ranges created thereby.

It is worth noting that the utilization of cotton with absorbent articles can be far from trivial. Cotton as it is harvested is generally hydrophobic which means that it does not absorb fluid very well. Cotton as harvested comprises oils and waxes on the outer surface of the fibers. As such, in order for the cotton to become hydrophilic, the cotton must be processed to remove these oils and waxes.

In some forms, the cotton fiber length in the at least one of the primary absorbent member 16, the secondary absorbent member 18, and/or the withdrawal member 20, may comprise a mean of about 12 mm or 13 mm. In contrast, staple fiber cotton ranges from 18 mm to 33 mm in length. Where organic cotton is utilized, the length of the fibers may be about 19 mm.

Additionally, the cotton fibers utilized in the absorbent tampon may have a specific diameter to drive permeability, capillary suction, and expansion properties. In some forms, the diameter is from about 7 microns to about 30 microns.

For those forms where the secondary absorbent member 18 and/or the withdrawal member 20 comprise cotton fibers, additional treatment may need to be provided to the cotton fibers in these features. For example, as noted above, while harvested cotton is generally hydrophobic, it is treated to make it more amenable for fluid absorbency. Additionally, this treatment also facilitates the further processing of the cotton, e.g. combing and laying. However, if a high percentage of cotton is utilized in the secondary absorbent member 18 and/or the withdrawal member 20, any fluid insult absorbed by the primary absorbent member 16 would be permitted -- and with gravity encouraged in some respects -- to migrate to the secondary absorbent member 18 and/or the withdrawal member 20. Such fluid migration can cause leakage and/or soiling of undergarments - neither of which is desirable.

As such, in some forms, the secondary absorbent member 18 and/or the withdrawal member 20 may be treated with a hydrophobic coating. Some suitable examples include hydrogenated cotton seed oil.

In some forms, the cotton utilized in the primary absorbent member 16, the secondary absorbent member 18, and/or the withdrawal member 20 may be bleached. In such forms, the cotton fibers of the aforementioned members may be chlorine bleached. In other forms, the cotton fibers of the aforementioned members may be bleached via hydrogen peroxide. In such forms, the absorbent tampon may be chlorine free.

In addition to the above concerns when utilizing cotton in the absorbent tampon, there are additional processing items which should be taken into account. For example, the compression of a tampon pledget to form the absorbent tampon 14 can create some additional concerns. As noted previously in the context of absorbent tampons, cotton is not as efficient as rayon on a gram per gram of absorbency basis; therefore, more cotton is required to achieve the same level of absorbency as a lower mass of rayon. In order to accommodate the increased mass of cotton, a larger diameter applicator could be utilized; however, a larger diameter could negatively impact the comfort during insertion of the tampon. In contrast, a cotton pledget could be compressed to higher degree to ensure that the cotton tampon fit into an existing diameter applicator. However, higher compression can negatively impact the amount of expansion of the tampon and can negatively impact the force required to expel the tampon from the applicator.

Regarding the expansion of the tampon, a general discussion of the processing of a tampon pledget is warranted. The absorbent tampon 14, in processing, begins as a tampon pledget. An exemplary tampon pledget 200 is shown in Figures 2B-2D. As shown, the pledget 200 can be compressed into the absorbent tampon 14 (shown in Figure 2A) that can be, for example, a generally cylindrical configuration.

Referring to Figures 2B-2D, the pledget 200 may comprise two layers of absorbent material 212 and 214 that combine to define a pledget thickness T. Forms of the present invention are contemplated where any suitable number of layers may be utilized - for example, one layer, two layers, three layers, four layers, five layers, six layers, seven layers, and so on.

Regardless of whether one layer or multiple layers are utilized, forms of the present disclosure are contemplated where the fibers in the one or more layers may comprise fiber integration in a Z-direction. For example, a single layer may be integrated in the Z-direction via hydroentagling or needlepunching processes. As another example, multiple layers may be integrated in the Z-direction together or individually.

However, the method of fiber integration can impact the structure of the absorbent material of the pledget 200. For example, for absorbent material that is needle punched, a tampon pledget 200 comprising 700 gsm of absorbent material can take on a variety of different forms. For example, the absorbent material may be configured as one layer, two layers, or more layers. In contrast, where the tampon pledget 200 comprises 700 gsm of absorbent material - where the absorbent material is spunlaced (hydroentangled) -- more than one layer will likely be required. Spunlacing of absorbent material having a basis weight of 700 gsm would be difficult at best. In such forms, the absorbent material would comprise a plurality of layers each of which or at least a portion of which were spunlaced.

It is believed that the fiber integrated nonwoven material can create a balance between expansion potential and fluid absorption kinetics. The fiber integrated nonwoven material of the tampons of the present disclosure provide good fluid distribution properties along the MD direction allowing the bottom of the pledget to expand. It is believed that this expansion of the bottom of the absorbent tampon 14 (shown in Figure 2A) can help reduce the likelihood of bypass leakage. At the same time however, the fiber integrated nonwoven material of the tampons of the present invention may also provide good fluid distribution properties along the Z-direction such that the likelihood of kinetic leakage is also reduced. Fiber integration in the Z-direction along with additional benefits thereof is described in additional detail in U.S. Patent Application 2016/0015572 and U.S. Patent Application Serial No. 62/418496.

As noted previously, the absorbent material of the present invention may comprise one or more layers. In some forms, each of the plurality of nonwoven layers may have a basis weight of about 100 grams per square meter ("gsm"). In some forms, each of the plurality of fiber integrated nonwoven layers may be at a basis weight of greater than about 20 gsm, greater than about 30 gsm, greater than about 40 gsm, greater than about 50 gsm, greater than about 60 gsm, greater than about 70 gsm, greater than about 75 gsm, greater than about 80 gsm, greater than about 85 gsm, greater than about 90 gsm, greater than about 95 gsm, greater than about 100 gsm, greater than about 110 gsm, greater than about 120 gsm, greater than about 130 gsm, greater than about 140 gsm, greater than about 150 gsm, greater than about 160 gsm, greater than about 170 gsm, greater than about 180 gsm, greater than about 190 gsm, or less than about 200 gsm or any ranges encompassed by these values and/or any number within these values.

Regardless of whether the absorbent material comprises multiple layers or one layer, the cumulative basis weight of the absorbent material may be between about 200 gsm and 1300 gsm. For example, the cumulative basis weight of the absorbent material 12 may be greater than about 200 gsm, greater than about 300 gsm, greater than about 400 gsm, greater than about 500 gsm, greater than about 600 gsm, greater than about 700 gsm, greater than about 800 gsm, greater than about 900 gsm, greater than about 1000 gsm, greater than about 1100 gsm, or less than or equal to about 1200 gsm. Forms are contemplated wherein the cumulative basis weight of the absorbent material comprises a range encompassed by the values above and/or wherein the cumulative basis weight of the absorbent material is a number within the values provided above.

The skilled artisan should appreciate that the mass of absorbent material of the pledget may be any suitable shaped, size, material, or construction. While pledget 200 is shown having a generally rectangular shape, other shapes are possible, including, for example, chevron, trapezoidal, triangular, semi-circular, "bow-tie", cross, and H.

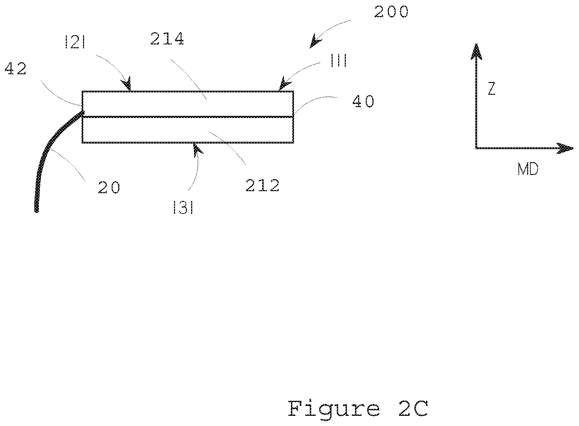

With regard to Figure 2C, a top 111 of the pledget 200 is disposed adjacent the leading end 40 and a bottom 121 of the pledget 200 is disposed adjacent the trailing end 42. A middle 131 is disposed between the top 111 and the bottom 121 of the pledget 10.

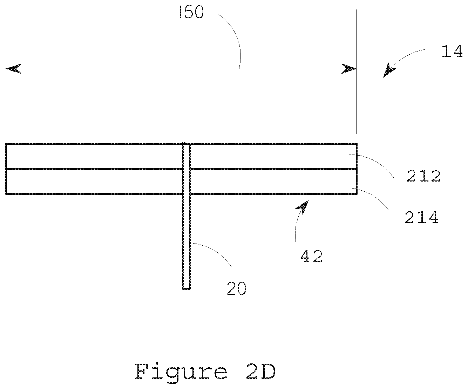

Tampon pledgets 200 of the present invention can be compressed into a generally cylindrical, self-sustaining form in the width direction, the radial direction, the axial direction, or any combination of these directions. Once compressed, the pledget 200 may be inserted into the applicator 12 (shown in Figure 1A). For the sake of clarity, the compressed tampon pledget 200 is referred to as the absorbent tampon 14 (shown in Figure 1A). Once inserted into the vagina, the absorbent tampon 14 (shown in Figure 1A) expands upon fluid contact. As shown in Figure 2D, the absorbent tampon 14 of the present disclosure expands upon fluid insult to eventually an expanded width 150. In order to reduce the likelihood of leakage, the absorbent tampon should expand as much as possible. Unfortunately, the level of compression of the pledget can negatively impact the amount of expansion that an absorbent tampon can exhibit.

However, despite the larger mass of cotton required, as described heretofore, the applicants have devised an absorbent tampon 14 derived from renewable resources which effectively expands upon fluid insult. In some forms, a tampon of the present invention which may be sold as a "Regular" absorbent tampon may comprise a mass of absorbent material which has been compressed into a generally cylindrical, self-sustaining form. The resulting tampon has an absorbent capacity as measured by the standard syngyna test of between about 6 to about 9 grams. The tampon is fluid expanding and preferably has an expanded width 150 upon fluid absorption of at least about 17 mm. Expanded widths are disclosed further in Tables 2 and 3 which show expanded widths for both Super and Regular absorbency tampons, respectively. Super absorbency and Regular absorbency are described below in additional detail.

Table 2

|  |  | Conventional Example #1 | Conventional Example #2 | Tampax Pearl Super | Sample #1 |
|---|---|---|---|---|---|
| Avg. Max Width @ 0g | mm | 15.10 | 15.60 | 15.38 | 15.8 |
| Avg. Max Width @ 3g | mm | 17.30 | 16.90 | 19.24 | 19.2 |
| Avg. Max Width @ 6g | mm | 17.90 | 17.40 | 21.02 | 20.5 |
| Avg. Max Width @ 9g | mm | 18.10 |  | 22.04 | 21 |
| Avg. Max Width Overall | mm | 18.20 | 17.60 | 22.49 | 21.1 |
| Avg. Max Width Growth Overall | mm | 3.10 | 2.00 | 7.11 | 5.2 |
| Avg. Max Length Overall | mm | 46.10 | 46.80 | 49.13 | 47.4 |

Table 3

|  |  | Conventional Example #3 | Conventional Example #4 | Tampax Pearl Regular | Sample #2 |
|---|---|---|---|---|---|
| Avg. Max Width @ 0g | mm | 12.90 | 14.00 | 13.57 | 14.2 |
| Avg. Max Width @ 3g | mm | 15.00 | 15.40 | 17.50 | 17.1 |
| Avg. Max Width @ 6g | mm | 15.40 | 16.00 | 19.01 | 18.2 |
| Avg. Max Width @ 9g | mm |  |  |  |  |
| Avg. Max Width Overall | mm | 15.40 | 16.10 | 19.44 | 18.3 |

(continued)

| | | Conventional Example #3 | Conventional Example #4 | Tampax Pearl Regular | Sample #2 |
|---|---|---|---|---|---|
| Avg. Max Width Growth Overall | mm | 2.50 | 2.20 | 5.87 | 4.1 |
| Avg. Max Length Overall | mm | 45.30 | 48.60 | 47.03 | 45.5 |

In some forms, a tampon of the present invention may be sold as a "Super" absorbency tampon. Such a tampon may comprise a mass of absorbent material which has been compressed into a generally cylindrical, self-sustaining form. The resulting tampon has an absorbent capacity as measured by the standard syngyna test of between about 9 to about 12 grams. The tampon is fluid expanding and preferably has an expanded width 150 upon fluid absorption of at least about 18 mm.

The increased mass of absorbent material within the tampons tested is shown in Table 4.

Table 4

| | Fiber (grams) | Overwrap (grams) | Total absorbent material (grams) | Total Tampon weight (grams) | Syngina absorbency (grams) | Bulk Density (grams/ml) |
|---|---|---|---|---|---|---|
| Sample #2 | 1.656 | 0.277 | 1.933 | 2.20 | 7.86 | 0.27 |
| Tampax Pearl Reg | 1.725 | Non absorbent | 1.725 | 2.20 | 7.92 | 0.31 |
| Conventional Example #3 | 1.45 | No overwrap | 1.45 | 1.60 | 7.32 | 0.24 |
| Conventional Example $4 | 2.0 | 0.1 | 2.1 | 2.5 | 7.13 | 0.24 |
| Sample #1 | 2.784 | 0.289 | 3.073 | 3.31 | 10.7 | 0.31 |
| Tampax Pearl Super | 2.544 | Non absorbent | 2.544 | 2.80 | 11.22 | 0.31 |
| Conventional Example #1 | 2.5 | No overwrap | 2.5 | 2.6 | 10.69 | 0.24 |
| Conventional Example #2 | 2.6 | 0.1 | 2.7 | 3.0 | 8.74 | 0.25 |

In addition to the expansion of the absorbent tampons described herein, the absorbent tampons are also housed within applicators that are not increased in diameter over what is currently available. As such, no additional discomfort is generated due to the increased mass of the absorbent tampon. In some forms, the insertion portion of the applicator has an outer diameter of 14 mm or less for a regular absorbency tampon and 16 mm or less for a super absorbency tampon.

The pledgets are compressed to a density ranging from about 0.25 to about 0.45 grams per cubic centimeter. The method for determining tampon density is provided below in the Test Methods section. In some forms, the pledgets are compressed to a density of greater than 0.27 grams per cubic centimeter, or a density of greater than 0.35 grams per cubic centimeter.

The setting of the compressed pledgets is believed to play a critical role in the expansion profile of the tampon pledget. Compressed tampon pledgets tend to re-expand to their original dimension which can greatly increase the expulsion force of the absorbent tampon from the insertion portion of the applicator. To overcome this tendency, heat-setting is utilized. However, thermal setting of compressed pledgets can lessen the absorptive capacity of the pledget if not done correctly. Some suitable methods of setting or stabilizing the tampon size and shape include heating a compressed pledget via microwaving as disclosed in U.S. Patent Application Serial Nos. 15/418032 and 15/159316. Preferably, the compressed pledget is subject to a conditioning via a microwave source for up to 25 seconds. The power of the microwave can be 2000-6000 Watts.

Forms of the present invention are also contemplated where the compressed absorbent pledgets are subjected to steam or thermal gradient conduction as described in U.S. Patent No. 7,047,608. Additional forms are contemplated where a heated gas or other medium can be applied to the compressed pledget via at least one pore or fluid communication passage while the compressed pledget is within a closed compression mold cavity.

Despite the increased mass of absorbent present in Sample #1 and Sample #2 versus the conventional examples nos. 1-4, the expulsion force is less in almost all instances versus the conventional examples. Expulsion force data is provided in Table 5 below.

Table 5

|  | Expulsion force (g) |
|---|---|
| Conventional Example #3 | 860 |
| Conventional Example #4 | 580 |
| Sample #2 | 614 |
| Tampax Pearl Reg | 432 |
| Conventional Example #1 | 900 |
| Conventional Example #2 | 840 |
| Sample #1 | 570 |
| Tampax Pearl Super | 850 |

For the data in Tables 1-4, Conventional Examples #1 and 3 are from Cora™ and are sold as organic tampons in Super absorbency and Regular absorbency, respectively. Conventional Examples #2 and 4 are from Kali™ and are sold as organic cotton tampons in Super absorbency and Regular absorbency, respectively. Tampax Pearl™ both Super and Regular absorbency are available in market.

Sample #1 was a super absorbency tampon constructed in accordance with the present description. Namely, the primary absorbent member comprises a 100% cotton and a 100% cotton overwrap. The applicator was created via bio-sourced polyethylene as described herein and comprised greater than about 90 percent bio-sourced polyethylene. Sample #2 was a regular absorbency tampon and constructed similar to Sample #1.

As discussed previously, the tampon pledget may be compressed; however, in some forms, the tampon pledget may be manipulated further prior to compression. For example, the mass of absorbent material may for example be rolled, folded, or otherwise altered in profile to help with compression and/or affect the expansion properties during use.

Once the compressed pledget is within an applicator, the tampon and applicator may be placed inside a wrapper. By 'wrapper material' it is meant herein any material suitable to be used for hygienically wrapping tampons and applicators. Said wrapper material has two surfaces; the 'inner surface' is directed towards the wrapped tampon and applicator, whereas the 'outer surface' is aligned opposite to said inner surface. Typically, suitable wrapper materials for use herein are flexible polymeric films, having a thickness of less than 1 mm. Examples for wrapper materials suitable for use are polymeric films made of polyethylene, polypropylene, polyester, cellophane, polyamide, poly(vinyl chloride), ethylene-vinyl acetate copolymer and the like. Alternatively, heat-shrinkable films, stretch films, pre-stretched elastic material, or combinations thereof may be used to create the wrapper. While not limited to a given composition, preferred compositions of heat-shrinkable and stretch films comprise primarily polyolefins such as polyethylene and polypropylene, or polyvinyl chloride. Polystyrene and polyethylene-terephtalate (PET), although being not heat sealable, are also suitable for use. Wrappers consisting of those materials can be closed by gluing with an adhesive. Other generally occlusive materials include metallic foils, such as aluminium foil. While occlusive wrapper materials are often preferred, in other situations non-occlusive or porous materials can be used, such as nonwovens, wovens, scrims, meshes and papers. Such non-occlusive materials can be made occlusive by combinations such as by lamination with or by coating with occlusive material. In the case of cellulosic papers, examples include lamination with a polymeric film such as a polyolefinic composition or coating or impregnation of the paper with wax. The aforementioned materials can be coated with various chemical compounds to improve their barrier properties or the ability for sealing. The wrapper may have a line of weakness or an improved opening means as described in U.S. Pat. No. 6,955, 665, filed May 23, 2002, entitled "Tampon Wrapper with Improved Opening Means," issued to Domeier, et al.; U.S. Pat. No. 8,302,844, filed November 20, 2006, entitled "Wrapper Having a Predetermined Line of Weakness," issued to Mc Connell, et al.; U.S. Patent No. 8,317,765; and U.S. Patent Application Publication No. 2003/0065300.

In some particular forms, the wrapper material may be derived from renewable resources. For example, in some forms, the wrapper material may be derived from a bio-based polyolefin film. In some forms, the wrapper may comprise a bio-based content value from about 10 percent to about 100 percent, from about 25 percent to about 90 percent, from about 40 percent to about 80 percent, or from about 50 percent to about 60 percent, specifically including all values within these ranges and any ranges created thereby.

Product Arrays

Forms of the present invention are contemplated where a single manufacturer offers an array of products to consumers. The single manufacturer may offer the array of products under a common brand identifier or brand name, in some forms. For example, a plurality of absorbent articles may be offered at least some of which utilize renewably resourced materials. In such forms, the array may comprise a first plurality of products and a second plurality of products. Each of the first plurality of absorbent articles may comprise first applicators and a first absorbent member within each of the first applicators. Each of the second plurality of absorbent articles may similarly comprise second applicators and a second absorbent member within each of the second applicators.

The first applicators may comprise materials which are substantially pertroleum derived, e.g. less than 10 percent bio-based content. In contrast, the second applicators may comprise materials which have a bio-based content of greater than 50 percent.

Additionally, the first absorbent members may comprise less than 50 percent renewably sourced materials. For example, the first absorbent members may comprise less than 50 percent cotton. In such forms, the first absorbent member may comprise any suitable absorbent material which at greater than about 50 percent by weight, e.g. rayon. Suitable absorbent materials are discussed in additional detail in U.S. Patent Application 2016/0015572 and U.S. Patent Application Serial No. 62/418496. In contrast, the second absorbent members may comprise greater than 50 percent renewably sourced material, e.g. cotton. For example, the second absorbent members may comprise greater than 50 percent cotton.

The first absorbent members may further comprise an overwrap which comprises less than 50 percent renewably sourced material. For example, a first overwrap, corresponding to each first absorbent member, may comprise more than 50 percent rayon. Suitable materials for overwraps are described in U.S. Patent Application 2016/0015572 and U.S. Patent Application Serial No. 62/418496. In contrast, the second absorbent members may further comprise an overwrap which comprises more than 50 percent renewably sourced material. For example, a second overwrap, corresponding to each second absorbent member may comprise more than 50 percent cotton.

The first absorbent member may further comprise a secondary absorbent member which comprises less than 50 percent renewably sourced material. For example, a secondary absorbent member associated with first absorbent members may comprise less than 50 percent cotton. In contrast, the second absorbent members may comprise a secondary absorbent member which comprises more than 50 percent renewably sourced material. For example, the secondary absorbent member associated with the second absorbent member may comprise more than 50 percent cotton. Suitable materials for the secondary absorbent material are described in U.S. Patent Application 2016/0015572 and U.S. Patent Application Serial No. 62/418496.

## TEST METHODS

Tampon Expulsion Force

The expulsion force can be measured by using a Tampon Expulsion Force Test. The Tampon Expulsion Force Test determines the force characteristics of tampon expulsion from an applicator. The test can utilize a constant rate of elongation tensile tester with a load cell chosen such that the measure force is between 10% and 90% of the cell's capacity. The test can also utilize an applicator fixture that must be able to hold applicator vertically downward (petals facing down) while exerting minimal force to the axial portion of the applicator.

The tester and fixture is placed in a room with a temperature of *21 °C (±4 °C).* This uniform temperature allows for comparison between products that expand based on temperature.

Sample preparation (if needed):

A test sample is prepared by removing any outer wrapper that may exist and tucking any out-hanging withdrawal cord inside the applicator pusher.

The applicator is placed into fixture such that the lip of the applicator is being held up (opposite to the direction of testing, preventing applicator from falling through the fixture). The fixture is then oriented within the tensile tester setup such that the direction of the force of the tensile tester should follow the long direction of the applicator and be applied directly on the pusher (plunger). The test is then run (speed: 1 mm/sec, data acquisition 20 hz) where the tensile tester is exerting downward force directly on the pusher such that the tampon moves toward and out of the petals. Test is complete when the body of the tampon has been completely expelled from the applicator.

Reporting of the results includes obtaining the force vs extension curves and determining the maximum expulsion force, as well as the number of peaks in the curve, at what displacements they occurred and the corresponding loads.

Tampon Density

The following steps are followed to calculate the density of a tampon according to the present invention. The tampon withdrawal string (or comparable withdrawal structure) and any existing secondary absorbent feature (e.g., a braid) is cut at the bottom of the tampon absorbent body. The tampon body is then weighed to the nearest 0.01 grams. And the tampon body length is measured to the nearest 0.1 millimeters. The tampon body is then immerse into isopropyl alcohol for 30 seconds or until air bubbles stop. Remove the tampon from the alcohol and allow the excess alcohol to drip from the tampon for15 seconds. Separately a test cylinder with spout is filled with isopropyl alcohol until it overflows at the spout into a 50 milliliter beaker. Tare the 50 milliliter beaker. Place the tampon body after the excess has dripped from the same slowly into the cylinder containing the standard volume of isopropyl alcohol. Collect the overflow of isopropyl alcohol from the spout that resulted from the addition of the tampon body. Obtain the weight of the isopropyl alcohol that overflowed the test cylinder to the nearest 0.01 grams.

$$\text{Tampon volume (milliliters)} = \text{weight of overflowed alcohol} / 0.780 \text{ grams per milliliter}$$

$$\text{Tampon density (grams per milliliter)} = \text{tampon weight (grams)} / \text{tampon volume}$$

Dynamic Expansion

Expanded Width is measured from the change in diameter of a tampon under pressure as test fluid is introduced at a specified flow rate. The expansion at three sites along the length of the tampon is measured at a specified fluid capacity and averaged to give the Expanded Width to the nearest 0.01 mm.

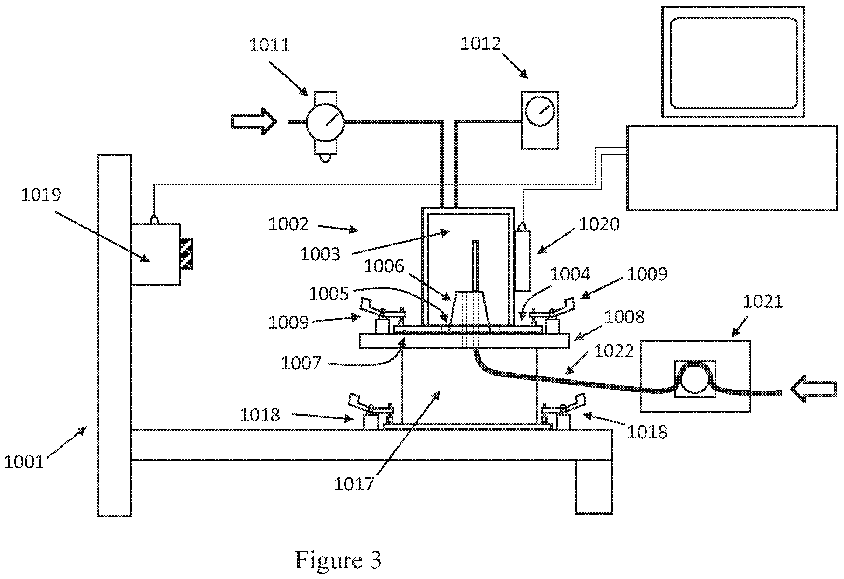

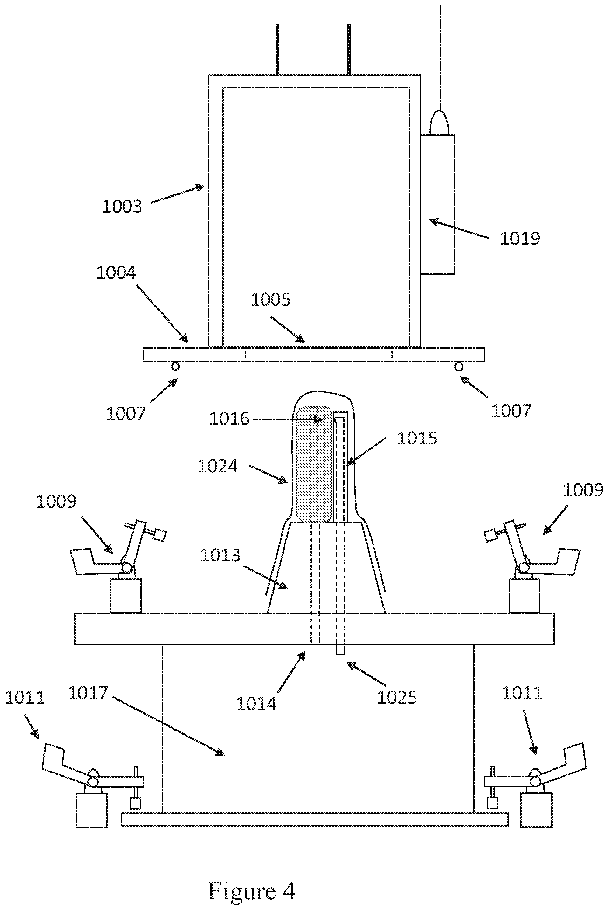

Referring to Figures 3 and Figure 4, the test apparatus includes a frame 1001 that supports the expansion assembly 1002. The assembly is constructed of a pressurized Plexiglas chamber 1003 with a bottom plate 1004. The plate has a circular opening 1005 such that the tampon mounting stand 1006 can be inserted through the opening. The bottom plate also has a silicone gasket 1007 that surrounds the opening 1005 and is used to seal the chamber 1003 against the mounting platform 1008 using clamps 1009. The chamber is pressurized to 3.45 kPa using a compressed air source 1010. The chamber pressure is adjusted via a pressure regulator 1011 and calibrated manometer 1012 (standardized to ANSI standards).

The tampon mounting stand 1006 is fixed to the mount plate 1008 and is made up of a tapered cylindrical base 1013 with a 3 mm diameter drain hole 1014 that extends from the top of the base and through the mount plate. A Plexiglas delivery tube 1015 (9.5 mm O.D by 3 mm I.D.) extends 48.5 mm above base 1013. The fluid path originates from a tube connector 1025 below the mount plate 1008 and extends through the mount base and terminates in a 1.5 mm opening 1016 on the side of the delivery tube 1015 (the top of the tube is sealed). The opening is 5 mm down from the top of the tube and faces the tampon. The mounting platform 1008 has a base 1017 that is open on two of its sides (to allow cleaning of fluid that drains from the drain hole 1014) and clamps 1018 to the frame 1001.

The imaging system consist of a sensor/camera 1019 (gray scale, minimum of 640 x 480 pixels, such as the Cognex DVT 545, available from PDF Supply, Cary NC, or equivalent) and is attached to the frame 1001 and light box 1020 (such as the Cognex DVT Smart Light, also available from PDF Supply, Cary NC, or equivalent) for backlighting is attached to the pressure chamber 1003. The camera is adjusted such that the total length of the sample is contained in the field of view. The system collects images and adjusts the threshold such that the sample in the foreground is dark against a light background. The digital images are analyzed using software suitable for making calibrated linear measurements. The software is calibrated for linear dimensions to the nearest 0.01 mm using standard cylinders of known diameters ranging from 12 to 24 mm. The horizontal width of the sample is measured at three sites; the "top" at 28% of the tampon length, "middle" at 50% of the tampon length, and "bottom" at 67% of the tampon length. All positions measured from the top of the tampon.

The test fluid used is Defibrinated Sheep's Blood, minimum 38% packed cell volume (available at Cleveland Scientific Ltd., Bath, OH, or equivalent). The test fluid is placed in a 250 mL reservoir and continuously and moderately stirred to avoid separation. The fluid is maintained at 23 °C $\pm$ 3 C° during use. The test fluid is supplied from the reservoir to the delivery tube 1015 with peristaltic pump tubing 2023. A peristaltic pump 1021 (such as Master Flex, available from Cole Parmer, Verner Hills, IL, or equivalent) delivers the test fluid at 1.0 g/min $\pm$ 0.02 g/min. The peristaltic pump tubing and delivery tube are filled before the start of testing.

Samples still in their applicators and wrappers are conditioned 23 °C $\pm$ 3 C° and 50 $\pm$ 5% relative humidity for 4 hours prior to testing. Samples are not removed from their wrappers or applicators until immediately prior to testing. Remove the tampon from its applicator and cut the withdrawal string at the base of the tampon. If a braid or skirt is present on the tampon, it is left intact. Measure the dry mass of the tampon to the nearest 0.01 gram. Using a digital Vernier caliper (e.g. Digimatic 500 series, Mitutoyo, or equivalent) measure the maximum length (not including skirt or braid if present) and maximum width and record each to the nearest 0.01 mm. This width is the Tampon Dry Width.

Place a small piece of 2-sided tape onto the sample side of the delivery tube 1015 making sure not to cover the side opening 1016. If the tampon's construction is a compressed, flat, sewn pad, mount the tampon so that the sewn side seam is toward the fluid delivery tube. For other constructions, direct any seam, if present, toward the delivery tube. Mount the sample onto the delivery tube with the top of the sample extending 2 mm above the top of the tube. If the sample length is greater than the height of the delivery tube, align the bottom of the sample to the top of the mounting base 1006. If the sample contains a braid or skirt, allow that structure to drape down the mounting base (opposite the delivery tube), assuring that it does not protrude past the width of the sample.

Unroll an unlubricated condom 2024 (condom compiling to ASTM 3492) and place loosely over the sample, delivery tube and upper portion of mounting base 1006 such that the end extends approximately 2 cm above the top of the sample. Place the expansion assembly over the sample and mounting base and clamp in place. Turn on the compressed air and adjust the pressure in the assembly to 3.45 kPa as read by the manometer. The condom should now be snug against the tampon. Start the vision system and inspect the resulting image. If folds of the condom appear at the sides or on top of sample in image, open chamber, readjust the condom, and repeat this step until the condom is smooth along the sides of the tampon in the image, e.g., no creases/wrinkles are visible.

Program the imaging system to take an image every 5 sec. Start the program and peristaltic pump. Continue flow to the sample until test fluid is detected at the bottom of the drain tube 1014. With the calibrated image software measure the width of the at the "top", "middle" and "bottom" sites for all images taken and record to the nearest 0.01 mm. Calculate the Corrected Widths as:

$$\text{Corrected Width}_{[s,i]} = \text{Dry Tampon Width} + \text{Width}_{(s,i)} - \text{Width}_{[s,0]}$$

Where s = site (Top, Middle, or Bottom); i = time (in sec); all widths in mm

From these values, separate curves for the Corrected Width (mm) vs Time (sec) can be constructed for each measured site.

Expanded Width is defined as the Corrected Width at a size-specific test mass. For each tampon select the Test Time for the appropriate absorbency term from Table 3. From each of the site Corrected Width vs Time curves read and record the Corrected Width for the "Top", "Middle" and "Bottom" sites at that selected Test Time. Calculate the arithmetic average of the three values and report as the Expanded Width to the nearest 0.01 mm.

In like fashion analyze 6 replicate samples, calculate the arithmetic average and report as the Expanded Width to the nearest 0.01 mm.

Table 3

| Absorbency Term | Absorbency Range (g) | Test Mass (g) | Test Time (s) |
|---|---|---|---|
| Light absorbency | 6 and under | 3.0 | 180 |
| Regular absorbency | 6 to 9 | 5.0 | 300 |
| Super absorbency | 9 to 12 | 8.0 | 480 |
| Super Plus absorbency | 12 to 15 | 11.0 | 660 |
| Ultra absorbency | 15 to 18 | 14.0 | 840 |
| No term | 18 and above | 17.0 | 1020 |

Validation of Polymers Derived from Renewable Resources

A suitable validation technique is through $^{14}$C analysis. A small amount of the carbon dioxide in the atmosphere is radioactive. This $^{14}$C carbon dioxide is created when nitrogen is struck by an ultra-violet light produced neutron, causing the nitrogen to lose a proton and form carbon of molecular weight 14 which is immediately oxidized to carbon dioxide. This radioactive isotope represents a small but measurable fraction of atmospheric carbon. Atmospheric carbon dioxide is cycled by green plants to make organic molecules during photosynthesis. The cycle is completed when the green plants or other forms of life metabolize the organic molecules, thereby producing carbon dioxide which is released back to the atmosphere. Virtually all forms of life on Earth depend on this green plant production of organic molecules to grow and reproduce. Therefore, the $^{14}$C that exists in the atmosphere becomes part of all life forms, and their biological products. In contrast, fossil fuel based carbon does not have the signature radiocarbon ratio of atmospheric carbon dioxide.

Assessment of the renewably based carbon in a material can be performed through standard test methods. Using radiocarbon and isotope ratio mass spectrometry analysis, the bio-based content of materials can be determined. ASTM International, formally known as the American Society for Testing and Materials, has established a standard method for assessing the bio-based content of materials. The ASTM method is designated ASTM D6866-10.

The application of ASTM D6866-10 to derive a "bio-based content" is built on the same concepts as radiocarbon dating, but without use of the age equations. The analysis is performed by deriving a ratio of the amount of organic radiocarbon ($^{14}$C) in an unknown sample to that of a modern reference standard. The ratio is reported as a percentage with the units "pMC" (percent modern carbon).

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It's gradually decreased over time with today's value being near 107.5 pMC. This means that a fresh biomass material such as corn could give a radiocarbon signature near 107.5 pMC.

Combining fossil carbon with present day carbon into a material will result in a dilution of the present day pMC content. By presuming 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, for example, it would give a radiocarbon signature near 54 pMC (assuming the petroleum derivatives have the same percentage of carbon as the soybeans).

A biomass content result is derived by assigning 100% equal to 107.5 pMC and 0% equal to 0 pMC. In this regard, a sample measuring 99 pMC will give an equivalent bio-based content value of 92%.

Assessment of the materials described herein can be done in accordance with ASTM D6866. The mean values quoted in this report encompasses an absolute range of 6% (plus and minus 3% on either side of the bio-based content value) to account for variations in end-component radiocarbon signatures. It is presumed that all materials are present day or fossil in origin and that the desired result is the amount of biobased component "present" in the material, not the amount of biobased material "used" in the manufacturing process.

Linear Distances

Linear distances may be measured by any appropriate instrument that is calibrated and capable of a measurement to the nearest 0.1 mm. Area measurements are made using the projected area of the article, as viewed orthogonally to the plane of the article length and width, in square millimeters to the nearest 0.1 mm$^2$. For the outer diameter of the insertion portion, measurement should be taken at the widest part of the insertion portion.

Fiber identification

The amount of cotton within the absorbent tampon can be determined via the method described from the American Association of Textile Chemists & Colorists under the name AATCC TM20A-2014.

Opacity

Opacity by contrast ratio measurements are made using a 0°/45° spectrophotometer suitable for making standard CIE L*a*b* color measurements (e.g. an X-Rite eXact spectrophotometer; available from X-Rite, Grand Rapids, MI or equivalent). The diameter of the instrument's measurement port should be chosen such that only the region of interest is included within the measurement port. A suitable diameter is 4 mm. Analyses are performed in a room controlled at about 23 °C $\pm$ 2 C° and 50 % $\pm$ 2 % relative humidity. Samples are conditioned at the same condition for 2 hours before testing.

Calibrate the instrument per the vender instructions using the standard targets provided by the vendor. Set the spectrophotometer to use the CIE XYZ color space, with a D65 standard illumination and 10° observer and nominal filter. A standard white target and black target, available from the vendor (e.g. standard opacity draw card BYK#2812 from X- Rite, or equivalent), is used as the backing for the opacity measurement.

Take the barrel portion of the applicator and cut across its diameter approximately 5 mm inboard of the tip of the barrel and 5 mm inboard of the grip of the barrel. Next take the barrel specimen and slice down its length such that the barrel can be unrolled into a planer specimen. Three opacity measurements, at sites equal distant along the length of the barrel, are taken for each applicator sample.

The outside surface of the barrel is placed facing the aperture of the instrument and is backed with the black target. The specimen is pressed flat and a CIE XYZ reading is taken and the Y value ($Y_{black}$) recorded to the nearest 0.01 units. In like fashion the specimen is measured in the same location using the white target as backing. The Y value ($Y_{white}$) is recorded to the nearest 0.01 units.

Opacity is calculated by dividing the $Y_{black}$ value measured using the black tile as backing, divided by the $Y_{white}$ value measured using the white tile as backing, then multiplying the ratio by 100. Record opacity to the nearest 0.01%. Calculate opacity for the 3 replicates and report the average opacity to the nearest 0.01%.

Fiber diameter

Traditionally cotton diameter is expressed in micronaire (actually a gravimetric fineness measurement) and measured as outlined in ASTM D1448-Standard Test Method for Micronaire Reading of Cotton Fibers. The units of micronaire are micrograms per inch and can then be converted to diameter through mathematical manipulation via the equations below.

$$Decitex = 0.393 * Micronaire$$

$$Denier\ (microns) = \sqrt{\frac{\left(\frac{4 * dtex}{Specific\ Gravity}\right)}{\pi}} * 10$$

where the specific gravity of cotton is 1.54 grams / cubic centimeter.

Fiber Length

The fiber length of the cotton utilized in the absorbent tampon of the present disclosure may be determined via ANSI/ASTM D1234 utilizing the L(n) methodology which measures the individual fiber length to create an average rather than a weighted average.

Syngyna

The Syngyna test is carried out as described in 21 CFR § 801.430(f)(2).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. An absorbent article comprising:

   a plastic applicator (12) comprising a bio-based content of 10 percent to 100 percent by weight using ASTM D6866-10, method B, the plastic applicator having an insertion portion (22) and a plunger portion (24), the plunger portion being movable within the insertion portion; and
   an absorbent tampon (14) disposed within the insertion portion, the absorbent tampon comprising a primary absorbent member (16) which is compressed to a density ranging from 0.25 to 0.45 grams per cubic centimeter as measured by the Tampon Density test method described herein, the primary absorbent member comprising from 50 percent to 100 percent by weight cotton fibers, wherein the plunger portion is capable of expelling the absorbent tampon from the insertion portion.

2. The absorbent article of claim 1, wherein the plastic applicator comprises a bio-based content preferably of 20 percent to 100 percent, more preferably from 30 percent to 95 percent, and most preferably from 75 percent to 90 percent using ASTM D6866-10, method B.

3. The absorbent article of any of the preceding claims, wherein the plastic applicator comprises a bio-based content of greater than 50 percent, preferably greater than 75 percent, more preferably greater than 80 percent, and most preferably greater than 90 percent using ASTM D6866-10, method B.

4. The absorbent article of any of the preceding claims, wherein the insertion portion comprises bio-based content of at least 95 percent and/or the plunger portion comprises a bio-based content of at least 95 percent using ASTM D6866-10, method B.

5. The absorbent article of any of the preceding claims, wherein the absorbent tampon comprises greater than 60 percent, preferably greater than 75 percent, more preferably greater than 90 percent, and most preferably greater than 95 percent cotton by weight.

6. The absorbent article of any of the preceding claims, wherein the absorbent tampon further comprises an overwrap which substantially covers the primary absorbent member, and wherein the overwrap comprises greater than 60 percent, preferably greater than 75 percent, more preferably greater than 90 percent, and most preferably greater than 95 percent cotton by weight and wherein the overwrap is spunlaced.

7. The absorbent article of any of the preceding claims, wherein the plastic applicator is substantially free of dyes and scents.

8. The absorbent article of any of the preceding claims, wherein the insertion portion has an outer diameter of 14 mm or less.

9. The absorbent article of any of the preceding claims, wherein the absorbent tampon has a density of between 0.27 grams / ml to 0.31 grams / ml.

10. The absorbent article of any of the preceding claims, wherein the absorbent tampon has an average dynamic expansion width of between 19.0 mm to 22.0 mm for a Super absorbency sized tampon or between 17.0 mm to 20.0 mm for a regular absorbency sized tampon, as measured with Dynamic Expansion test method described herein.

11. The absorbent article of any of the preceding claims, wherein the cotton fibers in the primary absorbent member have a mean length of less than 18 mm, preferably less than 16 mm, or more preferably less than 14 mm.

12. The absorbent article of any of the preceding claims, wherein the cotton fibers of the primary absorbent member comprise a mean length of 12 mm to 13 mm.

13. The absorbent article of any of the preceding claims, wherein the plunger portion comprises a first section and a second section, wherein the second section is slidably engaged with the first section, and wherein the second section is lockable with respect to the first section.

14. The absorbent article of any of the preceding claims, wherein the insertion portion has an outer diameter of 16 mm or less.

15. The absorbent article of any of the preceding claims, wherein the absorbent tampon further comprises a secondary absorbent member attached to the primary absorbent member, wherein the secondary absorbent member comprises polypropylene fibers and wherein the secondary absorbent member is sewn into the primary absorbent member.

16. A packaged absorbent article comprising the absorbent article of any of the preceding claims, and further comprising an outer wrapper, wherein the outer wrapper is derived from renewable polyethylene and exhibits a bio-based content of at least 50 percent to 100 percent using ASTM D6866-10, method B.

EP 3 681 453 B1

**Patentansprüche**

1. Absorbierendes Erzeugnis, umfassend:

   einen Kunststoffapplikator (12), umfassend einen biobasierten Gehalt von 10 Gewichtsprozent bis 100 Gewichtsprozent unter Verwendung von ASTM D6866-10, Verfahren B, wobei der Kunststoffapplikator einen Einführungsabschnitt (22) und einen Kolbenabschnitt (24) aufweist, wobei der Kolbenabschnitt innerhalb des Einführungsabschnitts bewegbar ist; und
   einen absorbierenden Tampon (14), der innerhalb des Einführungsabschnitts angeordnet ist, der absorbierende Tampon umfassend ein primäres absorbierendes Element (16), das auf eine Dichte in einem Bereich von 0,25 bis 0,45 Gramm pro Kubikzentimeter komprimiert wird, gemessen durch das hierin beschriebene Tampondichteprüfverfahren, das primäre absorbierende Element umfassend von 50 Gewichtsprozent bis 100 Gewichtsprozent Baumwollfasern, wobei der Kolbenabschnitt in der Lage ist, den absorbierenden Tampon aus dem Einführungsabschnitt auszustoßen.

2. Absorbierendes Erzeugnis nach Anspruch 1, wobei der Kunststoffapplikator einen biobasierten Gehalt von vorzugsweise 20 Prozent bis 100 Prozent, mehr bevorzugt von 30 Prozent bis 95 Prozent und am meisten bevorzugt von 75 Prozent bis 90 Prozent unter Verwendung von ASTM D 6866-10, Verfahren B, umfasst.

3. Absorbierendes Erzeugnis nach einem der vorstehenden Ansprüche, wobei der Kunststoffapplikator einen biobasierten Gehalt von mehr als 50 Prozent, vorzugsweise mehr als 75 Prozent, mehr bevorzugt mehr als 80 Prozent und am meisten bevorzugt mehr als 90 Prozent unter Verwendung von ASTM D6866-10, Verfahren B, umfasst.

4. Absorbierendes Erzeugnis nach einem der vorstehenden Ansprüche, wobei der Einführungsabschnitt einen biobasierten Gehalt von mindestens 95 Prozent umfasst und/oder der Kolbenabschnitt einen biobasierten Gehalt von mindestens 95 Prozent unter Verwendung von ASTM D6866-10, Verfahren B, umfasst.

5. Absorbierendes Erzeugnis nach einem der vorstehenden Ansprüche, wobei der absorbierende Tampon mehr als 60 Gewichtsprozent, vorzugsweise mehr als 75 Gewichtsprozent, mehr bevorzugt mehr als 90 Gewichtsprozent und am meisten bevorzugt mehr als 95 Gewichtsprozent Baumwolle umfasst.

6. Absorbierendes Erzeugnis nach einem der vorstehenden Ansprüche, wobei der absorbierende Tampon ferner eine Umwicklung umfasst, die im Wesentlichen das primäre absorbierende Element bedeckt, und wobei die Umwicklung mehr als 60 Gewichtsprozent, vorzugsweise mehr als 75 Gewichtsprozent, mehr bevorzugt mehr als 90 Gewichtsprozent und am meisten bevorzugt mehr als 95 Gewichtsprozent Baumwolle umfasst und wobei die Umwicklung wasserstrahlverfestigt ist.

7. Absorbierendes Erzeugnis nach einem der vorstehenden Ansprüche, wobei der Kunststoffapplikator im Wesentlichen frei von Farbstoffen und Duftstoffen ist.

8. Absorbierendes Erzeugnis nach einem der vorstehenden Ansprüche, wobei der Einführungsabschnitt einen Außendurchmesser von 14 mm oder weniger aufweist.

9. Absorbierendes Erzeugnis nach einem der vorstehenden Ansprüche, wobei der absorbierende Tampon eine Dichte von zwischen 0,27 Gramm/ml bis 0,31 Gramm/ml aufweist.

10. Absorbierendes Erzeugnis nach einem der vorstehenden Ansprüche, wobei der absorbierende Tampon eine durchschnittliche dynamische Ausdehnungsbreite von zwischen 19,0 mm bis 22,0 mm für einen Tampon mit Superabsorptionsvermögen oder zwischen 17,0 mm bis 20,0 mm für einen Tampon mit normalem Absorptionsvermögen aufweist, gemessen mit dem hierin beschriebenen Prüfverfahren für dynamische Ausdehnung.

11. Absorbierendes Erzeugnis nach einem der vorstehenden Ansprüche, wobei die Baumwollfasern in dem primären absorbierenden Element eine mittlere Länge von weniger als 18 mm, vorzugsweise weniger als 16 mm oder mehr bevorzugt weniger als 14 mm aufweisen.

12. Absorbierendes Erzeugnis nach einem der vorstehenden Ansprüche, wobei die Baumwollfasern des primären absorbierenden Elements eine mittlere Länge von 12 mm bis 13 mm umfassen.

17

**13.** Absorbierendes Erzeugnis nach einem der vorstehenden Ansprüche, wobei der Kolbenabschnitt einen ersten Abschnitt und einen zweiten Abschnitt umfasst, wobei der zweite Abschnitt mit dem ersten Abschnitt verschiebbar in Eingriff steht und wobei der zweite Abschnitt in Bezug auf den ersten Abschnitt verriegelbar ist.

**14.** Absorbierendes Erzeugnis nach einem der vorstehenden Ansprüche, wobei der Einführungsabschnitt einen Außendurchmesser von mindestens 16 mm aufweist.

**15.** Absorbierendes Erzeugnis nach einem der vorstehenden Ansprüche, wobei der absorbierende Tampon ferner ein sekundäres absorbierendes Element umfasst, das an dem primären absorbierenden Element angebracht ist, wobei das sekundäre absorbierende Element Polypropylenfasern umfasst und wobei das sekundäre absorbierende Element in das primäre absorbierende Element eingenäht ist.

**16.** Verpacktes absorbierendes Erzeugnis, umfassend das absorbierende Erzeugnis nach einem der vorstehenden Ansprüche und ferner umfassend eine Umverpackung, wobei die Umverpackung von erneuerbarem Polyethylen stammt und einen biobasierten Gehalt von mindestens 50 Prozent bis 100 Prozent unter Verwendung von ASTM D6866-10, Verfahren B, vorzeigt.

**Revendications**

**1.** Article absorbant comprenant :

un applicateur en plastique (12) comprenant une teneur d'origine biologique de 10 pour cent à 100 pour cent en poids conformément à la méthode B de la norme ASTM D6866-10, l'applicateur en plastique ayant une partie d'insertion (22) et une partie de piston (24), la partie de piston étant mobile à l'intérieur de la partie d'insertion ; et

un tampon absorbant (14) disposé à l'intérieur de la partie d'insertion, le tampon absorbant comprenant un élément absorbant primaire (16) qui est comprimé à une densité allant de 0,25 à 0,45 gramme par centimètre cube telle que mesurée par le procédé de test de densité de tampon décrit ici, l'élément absorbant primaire comprenant de 50 pour cent à 100 pour cent en poids de fibres de coton, dans lequel la partie de piston est capable d'expulser le tampon absorbant de la partie d'insertion.

**2.** Article absorbant selon la revendication 1, dans lequel l'applicateur en plastique comprend une teneur d'origine biologique de préférence de 20 pour cent à 100 pour cent, plus préférablement de 30 pour cent à 95 pour cent, et le plus préférablement de 75 pour cent à 90 pour cent conformément à la méthode B de la norme ASTM D6866-10.

**3.** Article absorbant selon l'une quelconque des revendications précédentes, dans lequel l'applicateur en plastique comprend une teneur d'origine biologique supérieure à 50 pour cent, de préférence supérieure à 75 pour cent, plus préférablement supérieure à 80 pour cent, et le plus préférablement supérieure à 90 pour cent conformément à la méthode B de la norme ASTM D6866-10.

**4.** Article absorbant selon l'une quelconque des revendications précédentes, dans lequel la partie d'insertion comprend une teneur d'origine biologique d'au moins 95 pour cent et/ou la partie de piston comprend une teneur d'origine biologique d'au moins 95 pour cent conformément à la méthode B de la norme ASTM D6866-10.

**5.** Article absorbant selon l'une quelconque des revendications précédentes, dans lequel le tampon absorbant comprend plus de 60 pour cent, de préférence plus de 75 pour cent, plus préférablement plus de 90 pour cent, et le plus préférablement plus de 95 pour cent de coton en poids.

**6.** Article absorbant selon l'une quelconque des revendications précédentes, dans lequel le tampon absorbant comprend en outre une surenveloppe qui recouvre essentiellement l'élément absorbant primaire, et dans lequel la surenveloppe comprend plus de 60 pour cent, de préférence plus de 75 pour cent, plus préférablement plus de 90 pour cent, et le plus préférablement plus de 95 pour cent de coton en poids et dans lequel la surenveloppe est lacée par filage.

**7.** Article absorbant selon l'une quelconque des revendications précédentes, dans lequel l'applicateur en plastique est essentiellement exempt de colorants et de parfums.

**8.** Article absorbant selon l'une quelconque des revendications précédentes, dans lequel la partie d'insertion a un diamètre de 14 mm ou moins.

**9.** Article absorbant selon l'une quelconque des revendications précédentes, dans lequel le tampon absorbant a une densité comprise entre 0,27 gramme/ml et 0,31 gramme/ml.

**10.** Article absorbant selon l'une quelconque des revendications précédentes, dans lequel le tampon absorbant a une largeur moyenne d'expansion dynamique comprise entre 19,0 mm et 22,0 mm pour un tampon de taille super absorbance ou entre 17,0 mm à 20,0 mm pour un tampon de taille absorbance régulière, telle que mesurée avec le procédé de test d'expansion dynamique décrit ici.

**11.** Article absorbant selon l'une quelconque des revendications précédentes, dans lequel les fibres de coton dans l'élément absorbant primaire ont une longueur moyenne inférieure à 18 mm, de préférence inférieure à 16 mm, ou plus préférablement inférieure à 14 mm.

**12.** Article absorbant selon l'une quelconque des revendications précédentes, dans lequel les fibres de coton de l'élément absorbant primaire comprennent une longueur moyenne de 12 mm à 13 mm.

**13.** Article absorbant selon l'une quelconque des revendications précédentes, dans lequel la partie de piston comprend une première section et une seconde section, dans lequel la seconde section est en prise de manière coulissante avec la première section, et dans lequel la seconde section est verrouillable par rapport à la première section.

**14.** Article absorbant selon l'une quelconque des revendications précédentes, dans lequel la partie d'insertion a un diamètre de 16 mm ou moins.

**15.** Article absorbant selon l'une quelconque des revendications précédentes, dans lequel le tampon absorbant comprend en outre un élément absorbant secondaire fixé à l'élément absorbant primaire, dans lequel l'élément absorbant secondaire comprend des fibres de polypropylène et dans lequel l'élément absorbant secondaire est cousu dans l'élément absorbant primaire.

**16.** Article absorbant emballé comprenant l'article absorbant selon l'une quelconque des revendications précédentes, et comprenant en outre une enveloppe externe, dans lequel l'enveloppe externe dérive de polyéthylène renouvelable et présente une teneur d'origine biologique d'au moins 50 pour cent à 100 pour cent conformément à la méthode B de la norme ASTM D6866-10.

Figure 1A

Figure 1B

EP 3 681 453 B1

Figure 2A

Figure 2B

Figure 2C

Figure 2D

Figure 3

EP 3 681 453 B1

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8449491 B
- US 8303558 B
- US 7081110 B
- US 8449 A
- US 491 A
- US 8075512 B
- US 20170020743 A
- US 6840927 B, Hasse
- US 3025585 A
- US 3949127 A
- US 4588360 A
- US 5873868 A
- US 7994387 B, Minoguchi
- US 5628097 A
- US 5916661 A
- US 20160015572 A
- US 62418496
- US 418032
- US 15159316 B
- US 7047608 B
- US 6955 A
- US 665 A, Domeier
- US 8302844 B, Mc Connell
- US 8317765 B
- US 20030065300